# EUROPÄISCHE PATENTSCHRIFT

(11) **EP 1 034 810 B1**
(45) Veröffentlichungstag und Bekanntmachung des Hinweises auf die Patenterteilung: **27.07.2005**
(21) Anmeldenummer: 99122360.3
(22) Anmeldetag: 10.11.1999
(51) Int. Cl.: A61M 5/31

(54) **Spritze für medizinische Zwecke und Verfahren zu ihrer Montage**
Syringe for medical purposes and method for its assembly
Seringue médicale et son procédé d'assemblage

(30) Priorität: 05.03.1999 DE 19909824
(43) Veröffentlichungstag der Anmeldung: 13.09.2000
(73) Patentinhaber: Arzneimittel GmbH Apotheker Vetter & Co. Ravensburg, D-88212 Ravensburg (DE)
(72) Erfinder: Vetter, Udo J., 88214 Ravensburg (DE); Otto, Thomas, 88250 Weingarten (DE); Hund, Petra, 88276 Berg (DE)
(74) Vertreter: Dziewior, Joachim, Dipl.-Phys. Dr.

(56) Entgegenhaltungen:
- DE-A- 19 547 431
- DE-A- 19 956 243
- US-A- 4 986 818

## Beschreibung

Die Erfindung betrifft eine Spritze für medizinische Zwecke, mit einem am Spritzenzylinder angesetzten, durch eine Verschlußkappe, beispielsweise ein Tip-Cap, bis zum Gebrauch der Spritze verschlossenen Nadelansatzstück, das in Form eines Luer-Konus zum Anbringen einer Kanüle eingerichtet ist, wobei die Verschlußkappe gemeinsam mit dem Ende des Nadelansatzstücks von einer Sicherungskappe umschlossen und die Verschlußkappe in der Sicherungskappe form- und/oder kraftschlüssig gehalten ist, ferner mit einem Haltering, der in seiner auf das Nadelansatzstück aufgeschobenen Position am Nadelansatzstück fest einrastbar ist. Weiter betrifft die Erfindung ein Verfahren zur Montage einer derartigen Spritze.

Aus der EP 0 716 860 ist eine Spritze mit einer Spritzenendstückkappe bekannt, die aus einer Innen- und einer Außenkappe besteht und in einen Luer-Bund eingeschraubt ist. Auf die Endstückkappe und den Luer-Bund ist ein Etikett aufgeklebt, das die Trennstelle zwischen beiden überdeckt und beim Entfernen der Endstückkappe reißt.

Ferner ist aus der DE 195 37 163 eine Spritze bekannt, die sich in der Praxis zwar bestens bewährt hat, bei der jedoch die einzelnen Verschlußteile zunächst auf entsprechend dafür ausgelegten Maschinen behandelt, also insbesondere gewaschen, gegebenenfalls silikonisiert und autoklaviert werden müssen, um sie anschließend - unter strengen Reinraumbedingungen - dem weiteren Herstellungsprozeß zuzuführen, wo sie dann - auf einer Füllmaschine - aufgesetzt werden.

Der Erfindung liegt die Aufgabe zugrunde, eine Verbesserung des Verfahrensablaufs zu ermöglichen, insbesondere das vor der Abfüllung bzw. nach dem Waschprozeß der Spritze erfolgende Verschließen zu optimieren.

Diese Aufgabe wird in vorrichtungsmäßiger Hinsicht nach der Erfindung dadurch gelöst, daß die Sicherungskappe fest an einem Sicherungsring angeschlossen, von diesem jedoch abtrennbar ausgebildet ist, wobei der Sicherungsring über den Haltering fest am Nadelansatzstück gehalten ist und den Haltering umgreift.

Der durch die Erfindung erreichte Vorteil besteht im wesentlichen darin, daß die einzelnen Verschlußteile zunächst unabhängig vom übrigen Herstell- bzw. Montageprozeß in ihren gebrauchsfertigen Zustand vormontiert werden können, so daß sich deren spätere Verwendung zum Schließen der Spritze auf ein einfaches Aufsetzen der vormontierten Verschlußteile auf das Nadelansatzstück reduziert. Da die vormontierten Verschlußteile gleichwohl die üblichen Reinigungsprozesse durchlaufen und sodann in sterilem Zustand dem Herstellungsprozeß beigefügt werden können bzw. beigefügt sind, ist durch die infolge der Vormontage erfolgte Reduzierung der zu handhabenden Teile eine höhere Prozeßsicherheit und ein vereinfachter Prozeßablauf zu erreichen.

In bevorzugter Ausführungsform der Erfindung ist vorgesehen, daß die Sicherungskappe innenseitig mit zwei sich diametral gegenüberstehenden, radial nach innen vorstehenden Rippen versehen ist. Dadurch wird erreicht, daß die Verschlußkappe fest in der Sicherungskappe gehalten wird.

Hierzu ist es jedoch ebenso möglich, daß die Sicherungskappe innenseitig mit einer Ringschulter versehen ist, die die Verschlußkappe mit einem Ringvorsprung hintergreift.

Weiter ist es im Rahmen der Erfindung von Vorteil, wenn die Sicherungskappe mit wenigstens einem an der Umfangsfläche angeordneten Fenster versehen ist. Zum einen besteht damit die Möglichkeit, eine Sichtkontrolle auf das Vorhandensein der Verschlußkappe vorzunehmen. Im übrigen führen die nach innen vorstehenden Rippen zu einer Verformung der Verschlußkappe zum Fenster hin, so daß der Rand des Fensters eine den Halt der Verschlußkappe fördernde Friktion ausübt.

Nach einer ersten vorteilhaften Ausführungsform der Erfindung kann der Haltering in einer Ringnut des Sicherungsrings angeordnet sein.

Es besteht jedoch gleichfalls die Möglichkeit, daß der Sicherungsring an seiner Innenmantelfläche einen den Haltering hintergreifenden Rastvorsprung aufweist. Entscheidend ist allein, daß der Haltering und der Sicherungsring eine ausreichend feste Verbindung eingehen, so daß ein unbeabsichtigtes oder auch beabsichtigtes Lösen der beiden Teile voneinander nicht - oder zumindest nicht ohne erkennbare Spuren zu hinterlassen - möglich ist.

Um für das Ansetzen der Kanüle auch einen Schraubanschluß nach dem sog. Luer-Lock-System zur Verfügung zu haben, kann der Haltering einen zum Ende des Nadelansatzstücks hin weisenden und von diesem durch einen Spalt beabstandeten Kragen aufweisen, dessen Innenmantelfläche mit einem Gewinde versehen ist und einen Luer-Lock-Anschluß für die Kanüle bildet.

Die zum Gebrauch der Spritze auftrennbare Verbindung zwischen der Sicherungskappe und dem Sicherungsring ist so gestaltet, daß die Sicherungskappe und der Sicherungsring zwischen sich einen Ringspalt bilden und über mehrere Verbindungsstege miteinander verbunden sind, wobei zwischen jeweils zwei Verbindungsstegen wenigstens ein Distanzteil angeordnet ist, das an der Sicherungskappe oder am Sicherungsring fest angeschlossen ist und dessen freies Ende dem Sicherungsring oder der Sicherungskappe anliegt oder mit geringem Abstand gegenübersteht.

Zweckmäßigerweise sind hierbei die Verbindungsstege gleichmäßig über den Umfang verteilt angeordnet.

Weiter hat es sich als vorteilhaft erwiesen, wenn die Verbindungsstege sich zum Sicherungsring hin verjüngen.

Schließlich empfiehlt es sich für einen zuverlässigen Halt der Verschlußteile, daß das Nadelansatzstück ein Rastelement in Form einer Ringnut, -wulst oder -schulter für den Haltering aufweist, wenngleich es grundsätzlich auch möglich ist, den Haltering auf dem Nadelansatzstück festzukleben.

Die der Erfindung zugrunde liegende Aufgabe wird weiter gelöst durch ein Verfahren zur Montage einer Spritze der vorbeschriebenen Art, das dadurch gekennzeichnet ist, daß zur Vormontage der kanülenseitig angeordneten Verschlußteile zunächst auf einem Hilfskonus der Haltering aufgebracht, sodann die Verschlußkappe aufgesetzt und schließlich die Sicherungskappe mit dem Sicherungsring aufgesetzt werden, wobei der Sicherungsring am Haltering eingerastet wird. Hierdurch entsteht - abgekoppelt vom eigentlichen Herstellungsprozeß - ein vollständig vormontiertes Verschlußteil, das im Anschluß an die erforderlichen Reinigungs- und Sterilisierungsschritte als eine Einheit im weiteren Herstellungsprozeß eingesetzt werden kann.

Zweckmäßigerweise kann auch die Vormontage der Verschlußteile bereits unter aseptischen Bedingungen erfolgen.

Zur weiteren Handhabung empfiehlt es sich, daß die vormontierten Verschlußteile gereinigt und in geeignete Sterilbeutel verpackt und sterilisiert werden.

Der Reinigungsprozeß kann im montierten Zustand (Nachbehandlung der Verschlußteile) oder im unmontierten Zustand (Vorbehandlung der Einzelteile) durchgeführt werden.

Schließlich kann der weitere Verfahrensablauf so gestaltet sein, daß die im Sterilbeutel verpackten Verschlußteile nach der Sterilisation über ein Schleusensystem dem weiteren, unter Reinraumbedingungen ablaufenden Produktionsgang zum Verschließen der Spritzenzylinder zugeführt oder die nicht autoklavierten Teile nach dem Waschprozeß montiert und mit der leeren, gewaschenen Spritze autoklaviert werden.

Im folgenden wird die Erfindung an in der Zeichnung dargestellten Ausführungsbeispielen näher erläutert; es zeigen:
- Fig. 1: in den Teilfiguren a) bis c) die aus der Sicherungskappe mit Sicherungsring, der Verschlußkappe und dem Haltering bestehenden Verschlußteile vor ihrer Vormontage,
- Fig. 2: den Gegenstand nach Fig. 1, jedoch in montiertem Zustand,
- Fig. 3: das kanülenseitige Ende des im übrigen nur angedeuteten Spritzenzylinders,
- Fig. 4: eine der Fig. 2 entsprechende Darstellung einer zweiten Ausführungsform,
- Fig. 5: eine Seitenansicht des Gegenstands nach Fig. 4,
- Fig. 6: einen Schnitt durch den Gegenstand nach Fig. 4 längs der Linie VI-VI,
- Fig. 7: und 8 eine den Fig. 2 und 3 entsprechende Darstellung der zweiten Ausführungsform nach Fig. 4.

Die in der Zeichnung nur angedeutete Spritze ist insbesondere für medizinische Zwecke vorgesehen und kann bezüglich ihrer allgemeinen Gestaltung weitgehend beliebig ausgebildet sein. Sie weist ein am Spritzenzylinder 1 angesetztes, durch eine Verschlußkappe 2, beispielsweise ein Tip-Cap, bis zum Gebrauch der Spritze verschlossenes Nadelansatzstück 3 auf. Dieses Nadelansatzstück 3 ist in Form eines Luer-Konus ausgebildet und zum Anbringen einer Kanüle eingerichtet. Die Verschlußkappe 2 ist gemeinsam mit dem Ende des Nadelansatzstücks 3 von einer Sicherungskappe 4 umschlossen, die über einen Sicherungsring 5 fest mit dem Nadelansatzstück 3 verbunden ist. Für den Gebrauch der Spritze ist die Sicherungskappe 4 jedoch vom Sicherungsring 5 abtrennbar ausgebildet.

Im Herstellungsprozeß werden die eben genannten, gemeinsam ein Verschlußteil bildenden Elemente entweder zunächst auf entsprechend dafür ausgelegten Maschinen vorbehandelt, also insbesondere gewaschen und gegebenenfalls silikonisiert, um sie anschließend - nach bzw. vor Sterilisierung und unter Reinraumbedingungen - dem weiteren Herstellungsprozeß zuzuführen oder die gemeinsam ein Verschlußteil bildenden Teile werden als Ganzes gewaschen. Dort werden sie dann - auf einer Füllmaschine bzw. Waschmaschine - im Anschluß an die Befüllung bzw. Reinigung der Spritze nacheinander einzeln aufgesetzt.

Dies macht - unter den genannten und somit kostenintensiven Reinraumbedingungen - mehrere Arbeitsschritte erforderlich, bis sämtliche der Verschlußteile montiert sind.

Um eine Verbesserung des Verfahrensablaufs zu ermöglichen, wird eine Spritze vorgeschlagen, bei der die Verschlußkappe 2 in der Sicherungskappe 4 form- und/oder kraftschlüssig gehalten ist, wobei der Sicherungsring 5 über einen Haltering 6 fest am Nadelansatzstück 3 angeschlossen ist. Der Sicherungsring 5 umgreift dabei den Haltering 6, wobei der Haltering 6 in seiner auf das Nadelansatzstück 3 aufgeschobenen Position am Nadelansatzstück 3 fest einrastbar ist.

Durch diese Gestaltung ist es möglich, die einzelnen Verschlußteile zunächst unabhängig vom übrigen Herstell- bzw. Montageprozeß der eigentliche Spritze in ihren gebrauchsfertigen Zustand vormontieren zu können. Dadurch reduziert sich - verfahrenstechnisch - deren spätere Verwendung beim Verschließen der Spritze auf ein einfaches Aufsetzen der vormontierten Verschlußteile auf das Nadelansatzstück 3.

Da die vormontierten Verschlußteile gleichwohl die üblichen Reinigungsprozesse durchlaufen und sodann in sterilem Zustand dem Herstellungsprozeß beigefügt werden können, wird eine höhere Prozeßsicherheit und ein vereinfachter Prozeßablauf erreicht, da infolge der Vormontage die Anzahl der beim Verschließen der Spritze zu handhabenden Teile erheblich, nämlich auf eins, reduziert wird.

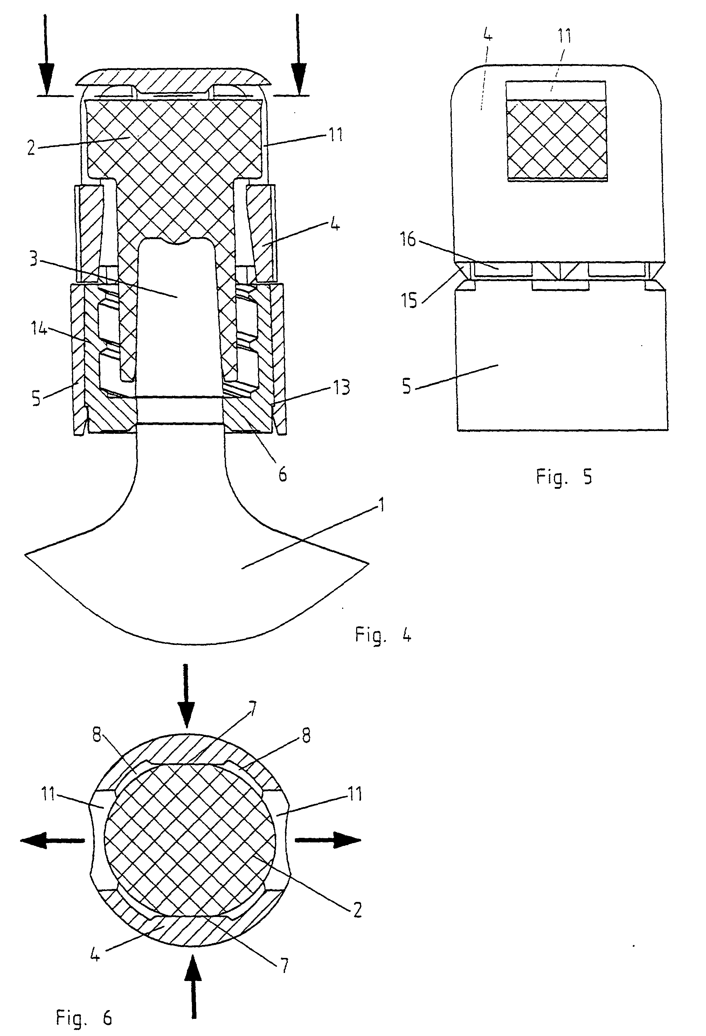

Wie sich aus Fig. 6 ergibt, sind an der Innenseite der Sicherungskappe 4 zwei sich diametral gegenüberstehende, radial nach innen vorstehende Rippen 7 vorgesehen, durch die erreicht wird, daß die Verschlußkappe 2 fest in der Sicherungskappe 4 gehalten wird. Zusätzlich werden seitlich der Rippen 7 Kanäle 8 geschaffen, die bei der Reinigung und Sterilisation den Eintritt von Flüssigkeit bzw. Dampf fördern.

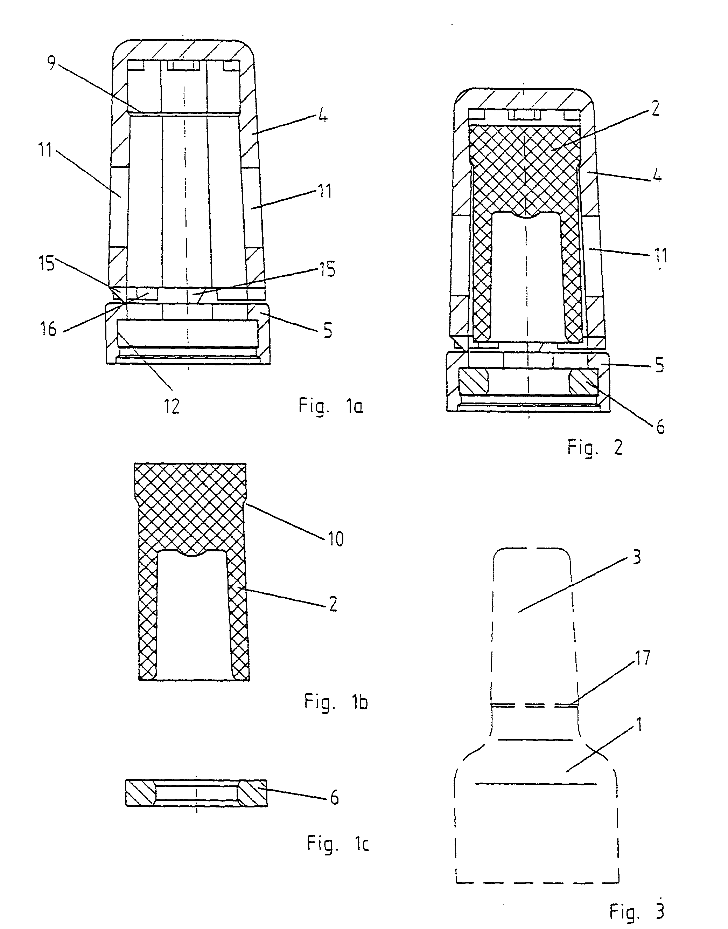

Gemäß der Figuren 1 und 2 ist es jedoch auch möglich, die Sicherungskappe 4 innenseitig mit einer Ringschulter 9 zu versehen, die die Verschlußkappe 2 mit einem Ringvorsprung 10 hintergreift. Auch hierdurch wird ein fester Halt der Verschlußkappe 2 in der Sicherungskappe 4 geschaffen.

Wie die Figuren 1, 2, 4 und 5 zeigen, ist die Sicherungskappe 4 mit jeweils zwei an der Umfangsfläche angeordneten Fenstern 11 versehen. Zum einen besteht damit die Möglichkeit, eine Sichtkontrolle auf das Vorhandensein der Verschlußkappe 2 vorzunehmen. Andererseits können auch die Fenster 11 den Eintritt von Flüssigkeit bzw. Dampf bei der Reinigung und Sterilisation fördern.

Im übrigen führen die nach innen vorstehenden Rippen 7 zu einer Verformung der Verschlußkappe 2 zum Fenster 11 hin, so daß der Rand des Fensters 11 auch den Halt der Verschlußkappe 2 durch Friktion fördern kann.

In der Ausführungsform nach den Fig. 1 und 2 ist der Haltering 6 in einer Ringnut 12 des Sicherungsrings 5 angeordnet.

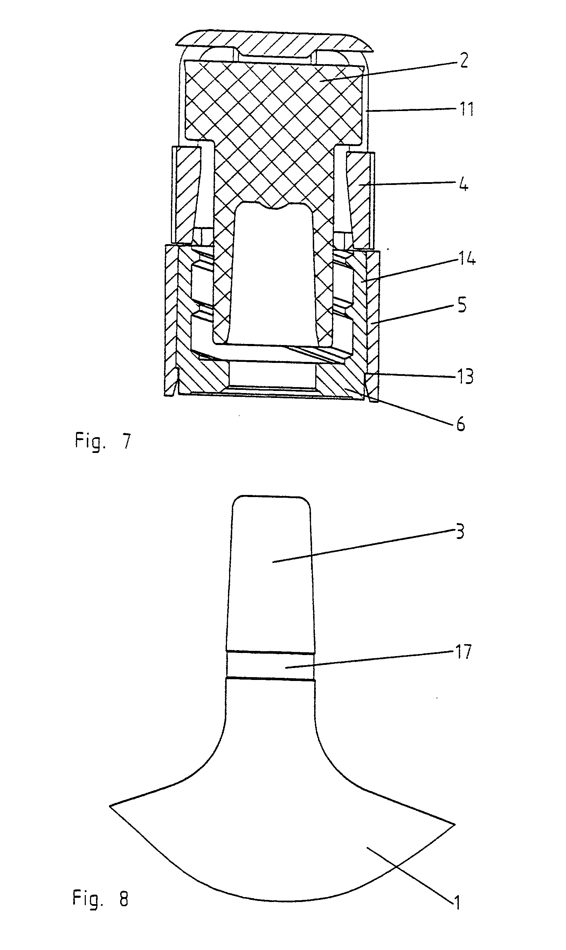

Bei der Ausführungsform nach den Fig. 4 bis 8 weist dagegen der Sicherungsring 5 an seiner Innenmantelfläche einen den Haltering 6 hintergreifenden Rastvorsprung 13 auf. Entscheidend ist jedoch - unabhängig von der Gestaltung im einzelnen - allein, daß der Haltering 6 und der Sicherungsring 5 eine ausreichend feste Verbindung eingehen. Dadurch ist ein unbeabsichtigtes oder auch beabsichtigtes Lösen der beiden Teile voneinander nicht möglich, jedenfalls nicht, ohne erkennbare Spuren zu hinterlassen.

Um für das Ansetzen der Kanüle auch einen Schraubanschluß nach dem sog. Luer-Lock-System zur Verfügung zu haben, weist der Haltering 6 gemäß der zweiten Ausführungsform nach den Fig. 4 bis 8 einen zum Ende des Nadelansatzstücks 3 hin weisenden und von diesem durch einen Spalt beabstandeten Kragen 14 auf. Dessen Innenmantelfläche ist mit einem Gewinde versehen, das einen Luer-Lock-Anschluß für die Kanüle bildet.

Die zum Gebrauch der Spritze auftrennbare Verbindung zwischen der Sicherungskappe 4 und dem Sicherungsring 5 ist in in der Zeichnung nicht näher dargestellter Weise so gestaltet, daß die Sicherungskappe 4 und der Sicherungsring 5 zwischen sich einen Ringspalt bilden und über mehrere Verbindungsstege 15 miteinander verbunden sind. Dabei ist zwischen jeweils zwei Verbindungsstegen 15 ein Distanzteil 16 angeordnet, das an der Sicherungskappe 4 oder am Sicherungsring 5 fest angeschlossen ist. Das freie Ende des Distanzteils 16 liegt dem Sicherungsring 5 oder der Sicherungskappe 4 entweder an oder steht ihr mit geringem Abstand gegenüber. Dadurch kann die Sicherungskappe 4 zusammen mit dem Sicherungsring 5 sowie der darin vormontierten Verschlußkappe 2 und dem Haltering 6 auf das Nadelansatzstück 3 aufgedrückt werden, ohne daß die Verbindungsstelle zwischen Sicherungskappe 4 und Sicherungsring 5 bricht oder beschädigt wird. Darüberhinaus verhindern die Distanzteile, daß die Sicherungskappe beim Aufsetzen über den Rastsitz hinausgeschoben wird.

Die Verbindungsstege 15 sind dabei üblicherweise gleichmäßig über den Umfang verteilt angeordnet und verjüngen sich zum Sicherungsring 5 hin.

Um eine sichere Befestigung des Halterings 6 am Nadelansatzstück 3 sicherzustellen, ist an diesem ein Rastelement 17 in Form einer Ringnut, -wulst oder -schulter für den Haltering 6 vorgesehen, an welchem der Haltering 6 einrastet.

Mit der vorstehend beschriebenen Spritze bzw. der Gestaltung der Verschlußteile läßt sich das Herstellungsverfahren insbesondere bei der Montage der Spritze wesentlich vereinfachen und verbessern, indem zur Vormontage der kanülenseitig vorgesehenen Verschlußteile der Spritze zunächst auf einem Hilfskonus der Haltering 6 aufgebracht, sodann die Verschlußkappe 2 aufgesetzt und schließlich die Sicherungskappe 4 mit dem Sicherungsring 5 aufgesetzt werden. Dabei wird der Sicherungsring 5 am Haltering 6 eingerastet, wodurch - abgekoppelt vom eigentlichen Herstellungsprozeß - ein vollständig vormontiertes Verschlußteil entsteht, das im Anschluß an die erforderlichen Reinigungs- und Sterilisierungsschritte als eine Einheit im weiteren Herstellungsprozeß eingesetzt werden kann.

Dabei kann auch bereits die Vormontage der Verschlußteile unter aseptischen Bedingungen erfolgen.

Zur weiteren Handhabung empfiehlt es sich, daß die vormontierten Verschlußteile in geeignete Sterilbeutel verpackt und sterilisiert werden.

Im einzelnen kann der weitere Verfahrensablauf so gestaltet sein, daß die im Sterilbeutel verpackten Verschlußteile nach der Sterilisation über ein Schleusensystem dem weiteren Produktionsgang zugeführt werden, wo dann unter Reinraumbedingungen die Spritzenzylinder nach Ihrer Befüllung verschlossen werden.

## Patentansprüche

1. Spritze für medizinische Zwecke, mit einem am Spritzenzylinder (1) angesetzten, durch eine Verschlußkappe (2), beispielsweise ein Tip-Cap, bis zum Gebrauch der Spritze verschlossenen Nadelansatzstück (3), das in Form eines Luer-Konus zum Anbringen einer Kanüle eingerichtet ist, wobei die Verschlußkappe (2) gemeinsam mit dem Ende des Nadelansatzstücks (3) von einer Sicherungskappe (4) umschlossen und die Verschlußkappe (2) in der Sicherungskappe (4) form- und/oder kraftschlüssig gehalten ist, ferner mit einem Haltering (6), der in seiner auf das Nadelansatzstück (3) aufgeschobenen Position am Nadelansatzstück (3) fest einrastbar ist, **dadurch gekennzeichnet, daß** die Sicherungskappe (4) fest an einem Sicherungsring (5) angeschlossen, von diesem jedoch abtrennbar ausgebildet ist, wobei der Sicherungsring (5) über den Haltering (6) fest am Nadelansatzstück (3) gehalten ist und den Haltering (6) umgreift.

2. Spritze nach Anspruch 1, **dadurch gekennzeichnet, daß** die Sicherungskappe (4) innenseitig mit zwei sich diametral gegenüberstehenden, radial nach innen vorstehenden Rippen (7) versehen ist.

3. Spritze nach Anspruch 1, **dadurch gekennzeichnet, daß** die Sicherungskappe (4) innenseitig mit einer Ringschulter (9) versehen ist, die die Verschlußkappe (2) mit einem Ringvorsprung (10) hintergreift.

4. Spritze nach einem der Ansprüche 1 bis 3, **dadurch gekennzeichnet, daß** die Sicherungskappe (4) mit wenigstens einem an der Umfangsfläche angeordneten Fenster (11) versehen ist.

5. Spritze nach einem der Ansprüche 1 bis 4, **dadurch gekennzeichnet, daß** der Haltering (6) in einer Ringnut (12) des Sicherungsrings (5) angeordnet ist.

6. Spritze nach einem der Ansprüche 1 bis 4, **dadurch gekennzeichnet, daß** der Sicherungsring (5) an seiner Innenmantelfläche einen den Haltering (6) hintergreifenden Rastvorsprung (13) aufweist.

7. Spritze nach einem der Ansprüche 1 bis 6, **dadurch gekennzeichnet, daß** der Haltering (6) einen zum Ende des Nadelansatzstücks (3) hin weisenden und von diesem durch einen Spalt beabstandeten Kragen (14) aufweist, dessen Innenmantelfläche mit einem Gewinde versehen ist und einen Luer-Lock-Anschluß für die Kanüle bildet.

8. Spritze nach einem der Ansprüche 1 bis 7, **dadurch gekennzeichnet, daß** die Sicherungskappe (4) und der Sicherungsring (5) zwischen sich einen Ringspalt bilden und über mehrere Verbindungsstege (15) miteinander verbunden sind, wobei zwischen jeweils zwei Verbindungsstegen (15) wenigstens ein Distanzteil (16) angeordnet ist, das an der Sicherungskappe (4) oder am Sicherungsring (5) fest angeschlossen ist und dessen freies Ende dem Sicherungsring (5) oder der Sicherungskappe (4) anliegt oder mit geringem Abstand gegenübersteht.

9. Spritze nach Anspruch 8, **dadurch gekennzeichnet, daß** die Verbindungsstege (15) gleichmäßig über den Umfang verteilt angeordnet sind.

10. Spritze nach Anspruch 8 oder 9, **dadurch gekennzeichnet, daß** die Verbindungsstege (15) sich zum Sicherungsring (5) hin verjüngen.

11. Spritze nach einem der Ansprüche 1 bis 10, **dadurch gekennzeichnet, daß** das Nadelansatzstück (3) ein Rastelement (17) in Form einer Ringnut, -wulst oder -schulter für den Haltering (6) aufweist.

12. Verfahren zur Montage einer Spritze nach den Ansprüchen 1 bis 11, **dadurch gekennzeichnet, daß** zur Vormontage der kanülenseitig angeordneten Verschlußteile zunächst auf einem Hilfskonus der Haltering aufgebracht, sodann die Verschlußkappe aufgesetzt und schließlich die Sicherungskappe mit dem Sicherungsring aufgesetzt werden, wobei der Sicherungsring am Haltering eingerastet wird.

13. Verfahren nach Anspruch 12, **dadurch gekennzeichnet, daß** die Vormontage der Verschlußteile unter aseptischen Bedingungen erfolgt.

14. Verfahren nach Anspruch 12 oder 13, **dadurch gekennzeichnet, daß** die vormontierten Verschlußteile in geeignete Sterilbeutel verpackt und sterilisiert werden.

15. Verfahren nach den Ansprüchen 12 bis 14, **dadurch gekennzeichnet, daß** die im Sterilbeutel verpackten Verschlußteile nach der Sterilisation über ein Schleusensystem dem weiteren, unter Reinraumbedingungen ablaufenden Produktionsgang zum Verschließen der Spritzenzylinder zugeführt werden.

## Claims

1. Syringe for medical purposes, with a needle hub (3) which is fitted onto the syringe barrel (1) and which until the syringe is used remains sealed by a sealing cap (2), for example a tip cap, and which is set up in the form of a Luer cone for fitting a cannula, wherein the sealing cap (2) together with the end of the needle hub (3) is encompassed by a security cap (4), and the sealing cap (2) is held in the security cap (4) in a form-fitting and/or non-positive manner, furthermore with a retaining ring (6) which, in its position as pushed on to the needle hub (3), can snap in firmly onto the needle hub (3), **characterised in that** the security cap (4) is firmly connected to a security ring (5), but is designed to be separable from it, wherein the security ring (5) is held firmly on the needle hub (3) via the retaining ring (6), and encompasses the retaining ring (6).

2. Syringe in accordance with claim 1, **characterised in that** the security cap (4) is equipped on the inside with two diametrically opposed ribs (7) that project radially inwards.

3. Syringe in accordance with claim 1, **characterised in that** the security cap (4) is equipped on the inside with an annular shoulder (9) which engages behind the sealing cap (2) with an annular projection (10).

4. Syringe in accordance with one of the claims 1 to 3, **characterised in that** the security cap (4) is equipped with at least one window (11) arranged on the peripheral surface.

5. Syringe in accordance with one of the claims 1 to 4, **characterised in that** the retaining ring (6) is arranged in an annular groove (12) of the security ring (5).

6. Syringe in accordance with one of the claims 1 to 4, **characterised in that** on its inner casing surface, the security ring (5) has a catch projection (13) that engages behind the retaining ring (6).

7. Syringe in accordance with one of the claims 1 to 6, **characterised in that** the retaining ring (6) has a collar (14) which points towards the end of the needle hub (3) and is separated from it by a gap, and whose inner casing surface is equipped with a screw thread and forms a Luer lock connection for the cannula.

8. Syringe in accordance with one of the claims 1 to 7, **characterised in that** the security cap (4) and the security ring (5) form between them an annular gap and are connected to one another via several connection fins (15), wherein arranged between two connection fins (15) in each case there is at least one spacer part (16) which is firmly connected to the security cap (4) or to the security ring (5) and whose free end rests against the security ring (5) or security cap (4) or stands opposite it at a small distance.

9. Syringe in accordance with claim 8, **characterised in that** the connection fins (15) are arranged distributed evenly over the periphery.

10. Syringe in accordance with claims 8 or 9, **characterised in that** the connection fins (15) taper towards the security ring (5).

11. Syringe in accordance with one of the claims 1 to 10, **characterised in that** the needle hub (3) has a catch element (17) in the form of an annular groove, annular ring or annular shoulder, for the retaining ring (6).

12. Method for assembling a syringe according to the claims 1 to 11, **characterised in that** for the pre-assembly of the sealing parts arranged on the cannula side, fitted on an auxiliary cone is first of all the retaining ring, then the sealing cap, and finally the security cap with the security ring, wherein the security ring is snapped onto the retaining ring.

13. Method according to claim 12, **characterised in that** the pre-assembly of the sealing parts takes place under aseptic conditions.

14. Method according to claim 12 or 13, **characterised in that** the pre-assembled sealing parts are packed in suitable sterile bags and are sterilised.

15. Method according to claims 12 to 14, **characterised in that** following sterilisation, the sealing parts packed in the sterile bag are sent on, via a lock system, to a further production cycle, which proceeds under clean room conditions, for sealing the syringe barrel.

## Revendications

1. Seringue à usage médical, comprenant un embout d'aiguille (3) monté sur le cylindre de seringue (1) et qui, jusqu'à utilisation de la seringue, est obturé par un capot de fermeture (2) par exemple un tip-cap, cet embout ayant la forme d'un cône de Luer pour montage d'une canule et ayant son extrémité entourée d'un capot de sécurité (4), dans lequel est maintenu le capot de fermeture (2) avec verrouillage par combinaison de formes et/ou par friction, ainsi qu'une bague de maintien (6) qui peut être bloquée fermement quand elle est dans sa position coulissée sur l'embout d'aiguille, **caractérisée en ce que** le capot de sécurité (4) est raccordé solidement àune bague de sécurité (5), mais toutefois est conçu pour pouvoir en être séparé, cette bague de sécurité (5) étant maintenue solidement sur l'embout d'aiguille (3) en entourant la bague de maintien (6).

2. Seringue selon la revendication 1, **caractérisée en ce que** le capot de sécurité (4) présente intérieurement deux nervures (7) en saillie radiale vers l'intérieur et diamétralement opposées.

3. Seringue selon la revendication 1, **caractérisée en ce que** le capot de sécurité (4) présente intérieurement un épaulement annulaire (9) qui est en prise par l'arrière avec le capot de fermeture (2) par l'intermédiaire d'une saillie annulaire (10).

4. Seringue selon une des revendications 1 à 3, **caractérisée en ce que** le capot de sécurité (4) présente au moins une fenêtre (11) dans sa surface périphérique.

5. Seringue selon une des revendications 1 à 4, **caractérisée en ce que** la bague de maintien (6) se trouve dans une rainure annulaire (12) de la bague de sécurité (5).

6. Seringue selon une des revendications 1 à 4, **caractérisée en ce que** la bague de sécurité (5) présente sur sa surface interne une saillie d'arrêt (13) en prise par l'arrière avec la bague de maintien (6).

7. Seringue selon une des revendications 1 à 6, **caractérisée en ce que** la bague de maintien (6) présente un collet (14) regardant vers l'extrémité de l'embout d'aiguille (4) et espacé de celui-ci par une fente, ce collet portant sur sa face interne un filetage et constituant un raccord du type Luer-lock pour la canule.

8. Seringue selon une des revendications 1 à 7, **caractérisée en ce que** le capot de sécurité (4) et la bague de sécurité (5) définissent entre eux une fente annulaire et sont reliés par plusieurs barrettes de liaison (15), avec chaque fois entre deux barrettes au moins une partie d'espacement (16) qui est raccordée solidement au capot de sécurité (4) ou à la bague de sécurité (5) et dont l'extrémité libre est en contact avec la bague de sécurité (5) ou le capot de sécurité (4), ou lui fait face à une faible distance.

9. Seringue selon la revendication 8, **caractérisée en ce que** les barrettes de liaison (15) sont réparties régulièrement à la périphérie.

10. Seringue selon la revendication 8 ou 9, **caractérisée en ce que** les barrettes de liaison (15) s'amincissent en direction de la bague de sécurité (5).

11. Seringue selon une des revendications 1 à 10, **caractérisée en ce que** l'embout d'aiguille (3) présente, pour la bague de maintien (6), un élément d'arrêt (17) en forme d'épaulement, de bourrelet ou de rainure annulaire.

12. Procédé pour le montage d'une seringue selon les revendications 1 à 11, **caractérisé en ce que** pour préassembler les parties de fermeture situées du côté de la canule, tout d'abord la bague de maintien est placée sur un cône auxiliaire, puis le capot de fermeture est posé, et enfin le capot de sécurité avec la bague de sécurité est monté et cette bague est bloquée sur la bague de maintien.

13. Procédé selon la revendication 12, **caractérisé en ce que** le préassemblage des parties de fermeture s'effectue dans des conditions aseptiques.

14. Procédé selon la revendication 12 ou 13, **caractérisé en ce que** les parties de fermeture préassemblées sont emballées dans une poche stérile appropriée et stérilisées.

15. Procédé selon les revendications 12 à 14, **caractérisé en ce que** les parties de fermeture emballées dans la poche stérile sont, après stérilisation, amenées à travers un système d'écluse à la phase suivante de la production qui s'effectue dans des conditions de salle blanche pour assurer la fermeture des cylindres de seringue.
